# EUROPEAN PATENT APPLICATION

(11) **EP 4 721 778 A1**
(43) Date of publication of application: **08.04.2026**
(21) Application number: 24823494.0
(22) Date of filing: 17.06.2024
(51) Int. Cl.: A61M 1/02

(54) **FILTER DEVICE**

(30) Priority: 16.06.2023 JP 2023099354
(71) Applicant: Terumo Kabushiki Kaisha, Tokyo 151-0072 (JP)
(72) Inventor: IIDA, Naoki, Fujinomiya-shi, Shizuoka 418-0004 (JP)
(74) Representative: TBK
(86) International application number: PCT/JP2024/021796
(87) International publication number: WO 2024/257881

(57) **Abstract**

A filter device (10) according to the present invention is to be mounted on a centrifugal separator (50) and includes: a first space (18) which is formed in a housing (12) and into which blood flows; a second space (20) which is formed in the housing (12) and from which blood flows out; a partition wall (22) that separates the first space and the second space and that is formed with a communication port (28) establishing communication between the first space and the second space; and a white-blood-cell removal filter (24) that is disposed in the second space. The first space is located between the partition wall and a first main surface (12a) of the housing, and the second space is located between the partition wall and a second main surface (12b) of the housing.

## Description

### Technical Field

The present invention relates to a filter device.

### Background Art

JP 5223006 B2 describes a blood bag system and a centrifugal transfer device (centrifugal separator). The blood bag system accommodates blood. The centrifugal separator centrifuges the blood accommodated in the blood bag system. The centrifuged blood is used for blood transfusion.

### Citation List

### Patent Literature

Patent Literature 1: JP 5223006 B2

### Summary of Invention

It is preferable that the blood to be transfused does not contain white blood cells. Recently, a filter device capable of more suitably removing white blood cells is desired.

It is therefore an object of the present invention to solve the above-described problem.

(1) One aspect of the present invention provides a filter device to be mounted on a centrifugal separator, the filter device including: a first space which is formed in a housing and into which blood flows through an inlet port; a second space which is formed in the housing and from which the blood flows out through an outlet port; a partition wall that separates the first space and the second space and that is formed with a communication port establishing communication between the first space and the second space; and a white-blood-cell removal filter that is disposed in the second space and that removes a white blood cell contained in the blood, wherein the first space is located between a first main surface of the housing and the partition wall formed along the first main surface, and the second space is located between a second main surface of the housing and the partition wall, the second main surface extending along the first main surface.

With this configuration, white blood cells can be suitably removed from blood.

(2) In the filter device according to (1), the inlet port may be located between a center line of the housing along a first direction and a rotation center of the centrifugal separator, the first direction intersecting a centrifugal direction in which a centrifugal force is applied.

With this configuration, the blood can smoothly flow into the housing by the centrifugal force.

(3) In the filter device according to (1) or (2), the first space may include a curved wall, and the curved wall may be located in a centrifugal direction in which a centrifugal force is applied relative to the inlet port and the communication port.

This configuration makes it possible to prevent backflow of blood toward the inlet port.

(4) In the filter device according to any one of (1) to (3), the first space may be provided with a meandering flow channel for allowing the blood flowing in through the inlet port to meander.

This configuration makes it possible to prevent blood from which white blood cells have not been removed by centrifugation from reaching the second space.

(5) In the filter device according to any one of (1) to (4), the communication port may be located on one side of the second space in a centrifugal direction in which a centrifugal force is applied, and the outlet port may be located on another side of the second space in the centrifugal direction.

With this configuration, a sufficient distance between the communication port and the outlet port in the centrifugal direction can be obtained.

(6) In the filter device according to (5), a direction from the outlet port toward the communication port may be a direction along the centrifugal direction.

This configuration makes it possible to prevent white blood cells from reaching the outlet port.

(7) In the filter device according to (5), a direction from the communication port toward the outlet port may be a direction along the centrifugal direction.

With this configuration, the blood can be suitably guided toward the outlet port by the centrifugal force.

(8) In the filter device according to any one of (1) to (7), a thickness direction may be along a vertical direction in a state where the filter device is mounted on the centrifugal separator.

With this configuration, the dimension of the filter device in the horizontal direction can be shortened as compared with the case where the first space and the second space are adjacent to each other in the horizontal direction.

According to the present invention, white blood cells can be suitably removed from blood.

### Brief Description of Drawings

Fig. 1 is a perspective view illustrating a centrifugal separator to which a filter device according to an embodiment is mounted.
Fig. 2 is a perspective view illustrating the filter device.
Fig. 3 is a cross-sectional view of the filter device.
Fig. 4 is a plan view illustrating a first space formed in a housing.
Fig. 5 is a plan view illustrating a second space formed in the housing.
Fig. 6 is a plan view illustrating a first space formed in a housing according to a first modification.
Fig. 7 is a plan view illustrating a second space formed in the housing according to the first modification.
Fig. 8 is a plan view illustrating a first space formed in a housing according to a second modification.

### Description of Embodiments

### [First Embodiment]

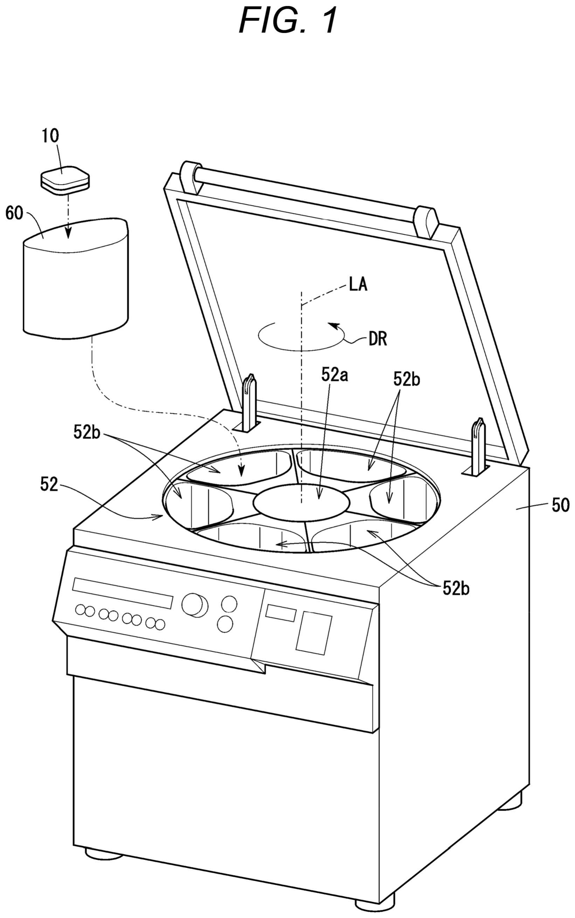

A filter device according to an embodiment will be described with reference to the drawings. Fig. 1 is a perspective view illustrating a centrifugal separator 50 to which a filter device 10 according to the present embodiment is mounted.

The centrifugal separator 50 is a machine that centrifugally separates blood. The centrifugal separator 50 includes a centrifugal drum 52. The centrifugal drum 52 includes a central body 52a and a plurality of unit insertion portions 52b.

The plurality of unit insertion portions 52b is disposed so as to surround the central body 52a. An insert unit 60 can be inserted into each of the plurality of unit insertion portions 52b. The insert unit 60 is attached to the centrifugal separator 50 by being inserted into the corresponding unit insertion portion 52b.

A blood bag system (not illustrated) is accommodated in the insert unit 60. The blood bag system includes a blood bag containing blood before being centrifuged. The blood contained in the blood bag is, for example, whole blood, but may be buffy coat as described in JP 5223006 B2 described above. The buffy coat contains red blood cells, platelets, white blood cells, and the like.

The centrifugal separator 50 rotates the insert unit 60 inserted into the unit insertion portion 52b about the central body 52a. More specifically, the centrifugal separator 50 rotates the insert unit 60 inserted into the unit insertion portion 52b along a rotation direction DR about the center line of rotation (rotation center) LA illustrated in Fig. 1. Accordingly, a centrifugal force is applied to the entire insert unit 60. The blood is centrifuged by the centrifugal force. The center line of rotation LA is, for example, along a gravity direction (second direction D2 to be described later).

The filter device 10 is mounted on the insert unit 60. The filter device 10 is mounted to, for example, but not limited to, an upper part of the insert unit 60.

The filter device 10 is mounted to the insert unit 60, and thus is mounted to the centrifugal separator 50 via the insert unit 60. The centrifugal force described above is applied not only to the insert unit 60 but also to the filter device 10. Note that the filter device 10 may be directly mounted to the centrifugal separator 50.

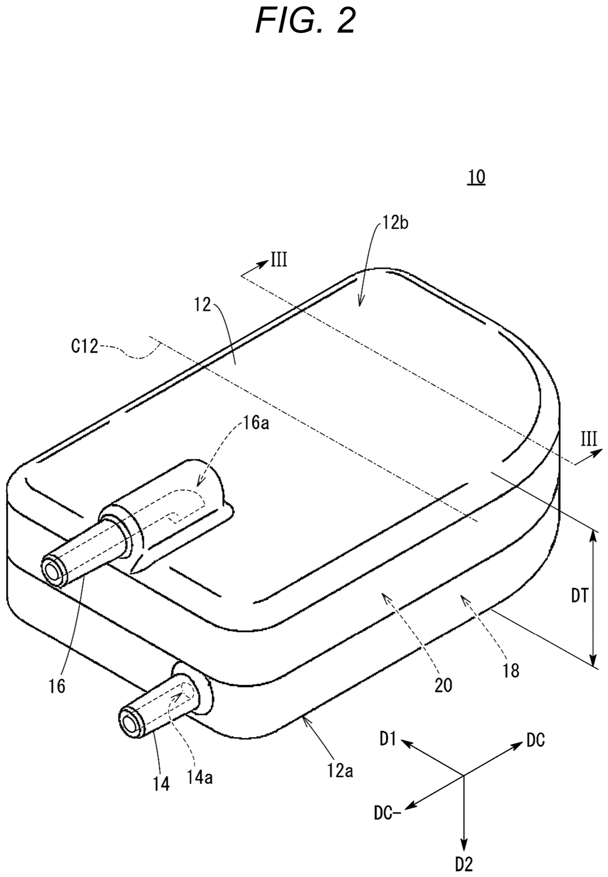

Fig. 2 is a perspective view illustrating the filter device 10.

As described above, the filter device 10 may be mounted to the centrifugal separator 50. In the following, the configuration of the filter device 10 will be described based on the premise that the filter device 10 is mounted to the centrifugal separator 50.

Fig. 2 illustrates a centrifugal direction DC, a centripetal direction DC-, a first direction D1, and a second direction D2. The centrifugal direction DC is a direction of the centrifugal force applied to the filter device 10 by the centrifugal separator 50. The first direction D1 is a direction of a tangential velocity in the circular movement of the filter device 10 by the centrifugal separator 50. The first direction D1 is orthogonal to (intersects) the centrifugal direction DC. The second direction D2 is a gravity direction (vertical direction). In the present embodiment, the centrifugal direction DC and the first direction D1 are orthogonal to the second direction D2. The centripetal direction DC- is a direction opposite to the centrifugal direction DC.

The filter device 10 includes a housing 12. The housing 12 can be formed in, for example, a box shape. In the present embodiment, in a state where the filter device 10 is mounted to the centrifugal separator 50, the thickness direction DT of the housing 12 coincides with (is parallel to) the second direction D2.

The housing 12 has a first main surface 12a and a second main surface 12b. The first main surface 12a is an outer surface on one side of the housing 12 in the thickness direction DT. The second main surface 12b is an outer surface on the other side of the housing 12 in the thickness direction DT. The first main surface 12a and the second main surface 12b extend along the centrifugal direction DC and the first direction D1.

In the present embodiment, the first main surface 12a is a lower surface of the housing 12. In the present embodiment, the second main surface 12b is an upper surface of the housing 12.

The housing 12 has an inlet portion 14. The inlet portion 14 has an inlet port 14a. The inlet port 14a is an opening that establishes communication between the inside and the outside of the housing 12. The inlet port 14a is located in the centripetal direction DC- relative to a center line C12 of the housing 12 along the first direction D1. In other words, the inlet port 14a is positioned between the center line C12 and the rotation center LA (Fig. 1) of the centrifugal separator 50. The inlet portion 14 illustrated in Fig. 2 protrudes from the housing 12, but is not limited thereto.

The housing 12 further has an outlet portion 16. The outlet portion 16 has an outlet port 16a. The inlet port 14a is an opening that establishes communication between the inside and the outside of the housing 12. The outlet portion 16 illustrated in Fig. 2 protrudes from the housing 12, but is not limited thereto.

Different blood bags are connected to the inlet portion 14 and the outlet portion 16. The blood bag connected to the inlet portion 14 is a blood bag containing, in advance, blood before being centrifuged. The blood bag connected to the outlet portion 16 is a blood bag for accommodating blood (blood component) that has passed through the filter device 10.

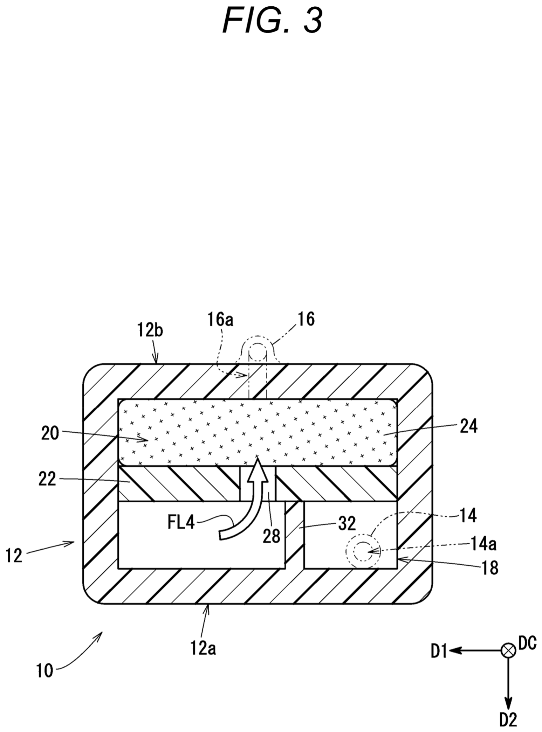

Fig. 3 is a cross-sectional view of the filter device 10. Fig. 3 illustrates a cross-sectional view taken along line III-III in Fig. 2.

The housing 12 has a first space 18, a second space 20, and a partition wall 22.

The first space 18 is a space located between the first main surface 12a and the partition wall 22. The first space 18 is in communication with the inlet port 14a. On the other hand, the second space 20 is a space located between the partition wall 22 and the second main surface 12b. The second space 20 is in communication with the outlet port 16a.

The partition wall 22 extends along the centrifugal direction DC and the first direction D1 similarly to the first main surface 12a, the second main surface 12b, and the like. The first space 18 and the second space 20 are separated by the partition wall 22. The partition wall 22 can define an upper surface of the first space 18 and a bottom surface of the second space 20.

The first space 18 is provided with an inhibition wall 32. The partition wall 22 is formed with a communication port 28. The communication port 28 and the inhibition wall 32 will be described later.

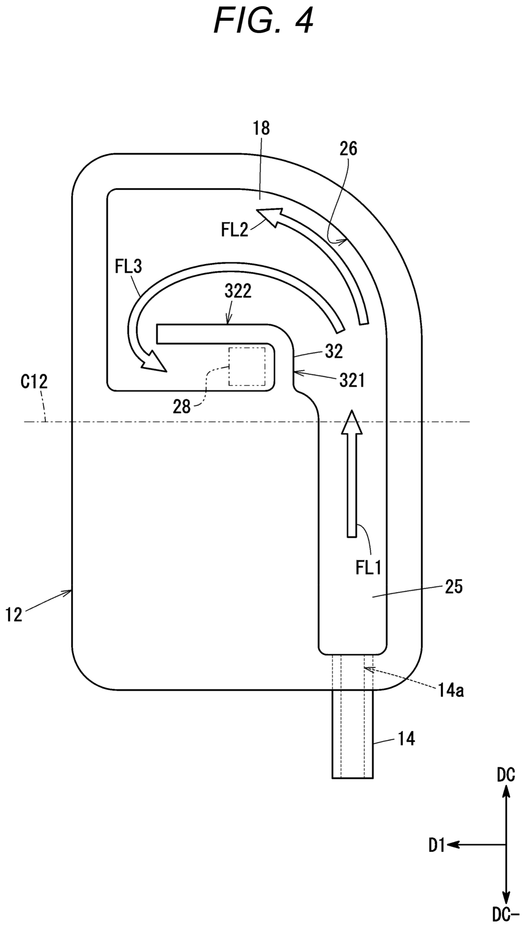

Fig. 4 is a plan view illustrating the first space 18 formed in the housing 12.

As described above, the first space 18 is in communication with the inlet port 14a. Therefore, blood flows into the first space 18 through the inlet port 14a.

The inlet port 14a is preferably formed along the centrifugal direction DC. In this case, the blood smoothly flows through the inlet port 14a and flows into the first space 18 by the centrifugal force applied by the centrifugal separator 50.

An inflow channel 25 can be formed in the first space 18. The inflow channel 25 is a flow path extending from the inlet port 14a along the centrifugal direction DC. The blood flowing into the first space 18 through the inlet port 14a can flow in the centrifugal direction DC along the inflow channel 25 (FL1).

The first space 18 may have a curved wall 26. The curved wall 26 is positioned in the centrifugal direction DC relative to the inlet port 14a and the communication port 28 described above. The curved wall 26 is curved so as to guide blood from one side to the other side of the first space 18 in the first direction D1. The blood flows along the curved wall 26. Therefore, the blood can flow from one side to the other side of the first space 18 in the first direction D1 without flowing back toward the inlet port 14a (FL2, FL3).

The blood flowing into the first space 18 is centrifuged by the centrifugal separator 50. As a result, the blood in the first space 18 has a layer of a supernatant liquid and a layer of a sedimentation liquid. The layer of the sedimentation liquid is located in the centrifugal direction DC relative to the layer of the supernatant liquid.

The main component of the sedimentation liquid is white blood cells. The reason why white blood cells are the main component of the sedimentation liquid is that white blood cells are relatively heavy blood components. That is, white blood cells are blood components that are relatively easily precipitated, and thus, are main components of the sedimentation liquid. On the other hand, the supernatant liquid contains a blood component lighter than white blood cells. For example, the supernatant liquid contains large quantities of platelets and the like.

The communication port 28 is an opening that establishes communication between the first space 18 and the second space 20. As described above, the communication port 28 is formed in the partition wall 22. The blood can flow from the first space 18 into the second space 20 through the communication port 28 (FL4 in Fig. 3).

The inhibition wall 32 inhibits the blood flowing into the first space 18 through the inlet port 14a from immediately reaching the communication port 28. In order for the blood to reach the communication port 28 from the inlet port 14a, the blood needs to bypass the inhibition wall 32. The inhibition wall 32 includes a first inhibition wall portion 321 and a second inhibition wall portion 322. The first inhibition wall portion 321 is a wall portion extending in the centrifugal direction DC. The first inhibition wall portion 321 is positioned between the communication port 28 and the inlet port 14a in the first direction D1. The second inhibition wall portion 322 is a wall portion extending in the first direction D1 from the first inhibition wall portion 321.

The second inhibition wall portion 322 illustrated in Fig. 4 protrudes in the first direction D1 from the leading end of the first inhibition wall portion 321, but is not limited thereto. The second inhibition wall portion 322 may not be provided.

Here, as described above, the white blood cells are collected on the side in the centrifugal direction DC in the first space 18 by the centrifugation with the centrifugal separator 50 (FL2). In other words, the white blood cells are less likely to flow in the centripetal direction DC- during the centrifugation with the centrifugal separator 50. Therefore, the white blood cells once flowing in the centrifugal direction DC from the communication port 28 are less likely to reach the communication port 28.

On the other hand, as described above, blood components other than the white blood cells can flow farther to the side in the centripetal direction DC- than the white blood cells due to the centrifugation with the centrifugal separator 50. Therefore, the blood components other than the white blood cells can reach the communication port 28 even after flowing in the centrifugal direction DC through the communication port 28 (FL3).

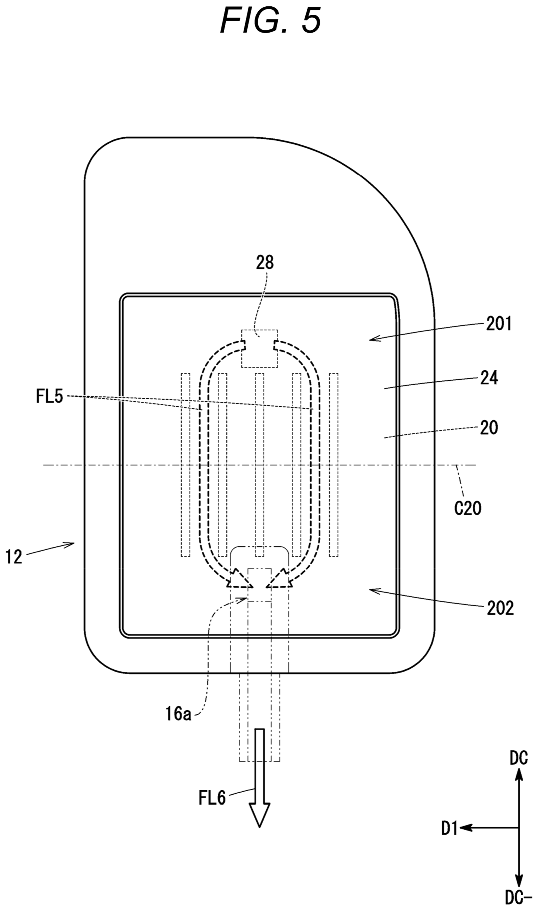

Fig. 5 is a plan view illustrating the second space 20 formed in the housing 12.

A white-blood-cell removal filter 24 is provided in the second space 20 (see also Fig. 3). The white-blood-cell removal filter 24 includes a filter medium for removing white blood cells from blood. The blood that has flowed into the second space 20 is filtered by the white-blood-cell removal filter 24 (FL5 in Fig. 5). Thus, even if white blood cells flow into the second space 20, the white blood cells are removed by the white-blood-cell removal filter 24.

As described above, the second space 20 is in communication with the outlet port 16a. Therefore, the blood in the second space 20 can flow out of the housing 12 through the outlet port 16a (FL6).

The second space 20 includes a first section 201 and a second section 202. The first section 201 is a region on one side of the second space 20 in the centrifugal direction DC. On the other hand, the second section 202 is a region on the other side of the second space 20 in the centrifugal direction DC. More specifically, the first section 201 is a region on the side in the centrifugal direction DC relative to the center line C20 in the second space 20. On the other hand, the second section 202 is a region on the side in the centripetal direction DC-relative to the center line C20 in the second space 20. The center line C20 is a virtual straight line that passes through the center of the second space 20 and is parallel to the first direction D1.

The communication port 28 is located in the first section 201. On the other hand, a communicating portion between the outlet port 16a and the second space 20 is located in the second section 202. With this configuration, a flow path of blood sufficient for removing white blood cells by the white-blood-cell removal filter 24 can be provided between the communication port 28 and the outlet port 16a. As a result, white blood cells contained in the blood can be more reliably removed by the white-blood-cell removal filter 24 before the blood reaches the outlet port 16a from the communication port 28.

In order for the blood flowing into the second space 20 to reach the outlet port 16a, the blood needs to move in the centripetal direction DC- from the communication port 28. As described above, the white blood cells are less likely to flow in the centripetal direction DC- during the centrifugation with the centrifugal separator 50. That is, since the direction from the outlet port 16a toward the communication port 28 is the centrifugal direction DC as illustrated in Fig. 5, it is possible to prevent white blood cells from reaching the outlet port 16a. Thus, the amount of white blood cells contained in the blood taken out from the outlet port 16a can be more suitably reduced.

As described above, according to the present embodiment, the blood flowing into the first space 18 is centrifuged by the centrifugal separator 50. Thus, the amount of white blood cells contained in the blood flowing into the second space 20 is reduced. The white blood cells remaining in the blood flowing into the second space 20 are removed by the white-blood-cell removal filter 24. That is, according to the present embodiment, the removal of white blood cells by centrifugation and the removal of white blood cells by the white-blood-cell removal filter 24 can be achieved on blood only by operating the centrifugal separator 50.

### [Modifications]

Modifications according to the above embodiment will be described below. Note that the description overlapping with the above embodiment will be appropriately omitted. Elements described in the above embodiment are denoted by the same reference numerals as those in the above embodiment unless otherwise specified.

### (First Modification)

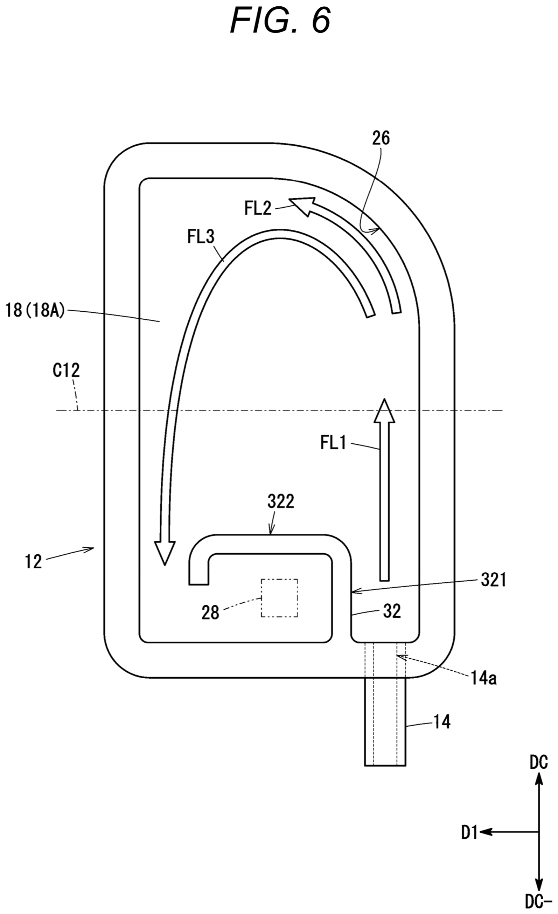

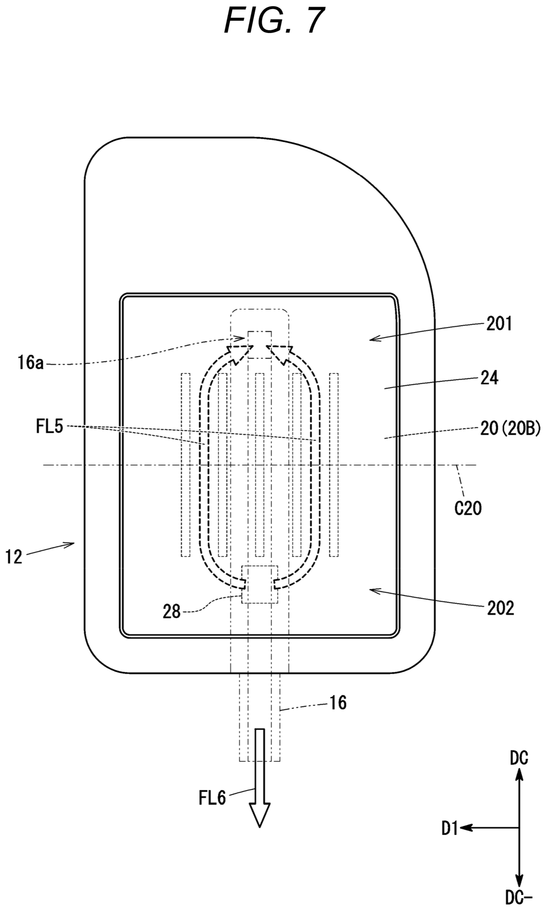

Fig. 6 is a plan view illustrating a first space 18 (18A) formed in a housing 12 according to the first modification. Fig. 7 is a plan view illustrating a second space 20 (20B) formed in the housing 12 according to the first modification.

In the embodiment, the communication port 28 is located on the side in the centrifugal direction DC relative to the center line C12 of the housing 12, but the present invention is not limited thereto. As illustrated in Fig. 6, the communication port 28 may be located on the side in the centripetal direction DC- relative to the center line C12. The communication port 28 is located as far as possible in the centripetal direction DC- in the first space 18A, by which it is possible to prevent white blood cells from reaching the communication port 28.

As illustrated in Fig. 7, the communication port 28 may be located in a second section 202 of the second space 20B. In a case where the communication port 28 is located in the second section 202 of the second space 20B, a communicating portion between the outlet port 16a and the second space 20B may be located in a first section 201. With this configuration, a flow path of blood sufficient for removing white blood cells by the white-blood-cell removal filter 24 can also be provided between the communication port 28 and the outlet port 16a.

In the present modification, the direction from the communication port 28 toward the outlet port 16a is along the centrifugal direction DC (FL5 in Fig. 7). Therefore, the blood flowing into the second space 20B can smoothly reach the outlet port 16a from the communication port 28 by the centrifugal force.

### (Second Modification)

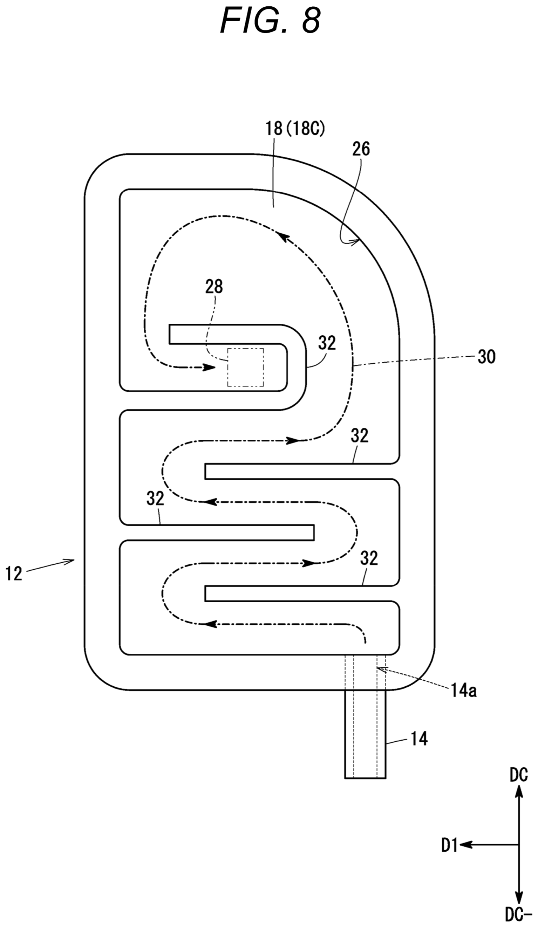

Fig. 8 is a plan view illustrating a first space 18 (18C) formed in a housing 12 according to the second modification.

As illustrated in Fig. 8, a meandering flow channel 30 for allowing blood flowing in through the inlet port 14a to meander is formed in the first space 18C. The meandering flow channel 30 may be constituted by, for example, a plurality of inhibition walls 32.

The blood flowing in through the inlet port 14a reaches the communication port 28 through the meandering flow channel 30. Thus, it is possible to prevent the blood from immediately flowing into the communication port 28 from the inlet port 14a. That is, it is possible to prevent blood from which white blood cells have not been removed by centrifugation from reaching the second space 20. The specific shape of the meandering flow channel 30 is not limited to the example in Fig. 8.

### (Combination of Multiple Modifications)

The plurality of modifications described above may be appropriately combined as long as there is no inconsistency.

Note that the present invention is not limited to the above disclosure and can take various configurations without departing from the gist of the present invention.

## Claims

1. A filter device to be mounted on a centrifugal separator, the filter device comprising:
a first space which is formed in a housing and into which blood flows through an inlet port;
a second space which is formed in the housing and from which the blood flows out through an outlet port;
a partition wall that separates the first space and the second space and that is formed with a communication port establishing communication between the first space and the second space; and
a white-blood-cell removal filter that is disposed in the second space and that removes a white blood cell contained in the blood, wherein
the first space is located between a first main surface of the housing and the partition wall formed along the first main surface, and
the second space is located between a second main surface of the housing and the partition wall, the second main surface extending along the first main surface.

2. The filter device according to claim 1, wherein
the inlet port is located between a center line of the housing along a first direction and a rotation center of the centrifugal separator, the first direction intersecting a centrifugal direction in which a centrifugal force is applied.

3. The filter device according to claim 1, wherein
the first space includes a curved wall, and
the curved wall is located in a centrifugal direction in which a centrifugal force is applied relative to the inlet port and the communication port.

4. The filter device according to claim 1, wherein
the first space is provided with a meandering flow channel for allowing the blood flowing in through the inlet port to meander.

5. The filter device according to claim 1, wherein
the communication port is located on one side of the second space in a centrifugal direction in which a centrifugal force is applied, and
the outlet port is located on another side of the second space in the centrifugal direction.

6. The filter device according to claim 5, wherein
a direction from the outlet port toward the communication port is a direction along the centrifugal direction.

7. The filter device according to claim 5, wherein
a direction from the communication port toward the outlet port is a direction along the centrifugal direction.

8. The filter device according to claim 1, wherein
a thickness direction is along a vertical direction in a state where the filter device is mounted on the centrifugal separator.
